# EUROPEAN PATENT SPECIFICATION

(11) **EP 3 633 608 B1**
(45) Date of publication and mention of the grant of the patent: **11.12.2024**
(21) Application number: 18809235.7
(22) Date of filing: 29.05.2018
(51) Int. Cl.: G06T 7/00, A61C 13/00, G16H 50/50

(54) **METHOD FOR DESIGNING A DENTAL PROSTHESIS USING DIGITAL TECHNIQUES**
VERFAHREN ZUM ENTWURF EINER ZAHNPROTHESE MITTELS DIGITALER TECHNIKEN
PROCÉDÉ DE CONCEPTION D'UNE PROTHÈSE DENTAIRE AU MOYEN DE TECHNIQUES NUMÉRIQUES

(30) Priority: 01.06.2017 ES 201730757
(43) Date of publication of application: 08.04.2020
(73) Proprietor: Laboratorios Pedro Perales, S.L.P., 23400 Ubeda Jaen (ES)
(72) Inventor: PERALES PULIDO, Diego, 41927 Mairena del Aljarafe (Sevilla) (ES); PERALES PADILLA, Pedro, 41927 Mairena del Aljarafe (Sevilla) (ES); PERALES PULIDO, Pedro, 41927 Mairena del Aljarafe (Sevilla) (ES)
(74) Representative: Capitán García, Maria Nuria
(86) International application number: PCT/ES2018/070388
(87) International publication number: WO 2018/220248

(56) References cited:
- EP-A1- 0 084 760
- EP-A2- 2 165 672
- WO-A1-2016/131939
- WO-A1-2017/044347
- WO-A1-2017/070358
- US-A1- 2012 259 597
- US-A1- 2015 025 855
- US-A1- 2015 173 870
- FRITS A. RANGEL ET AL: "Integration of Digital Dental Casts in Cone-Beam Computed Tomography Scans", ISRN DENTISTRY, vol. 2012, 1 January 2012 (2012-01-01), pages 1 - 6, XP055687914, DOI: 10.5402/2012/949086
- FRITS A. RANGEL ET AL: "Accuracy and Reliability of a Novel Method for Fusion of Digital Dental Casts and Cone Beam Computed Tomography Scans", PLOS ONE, vol. 8, no. 3, 20 March 2013 (2013-03-20), pages e59130, XP055687389, DOI: 10.1371/journal.pone.0059130
- SWENNEN ET AL: "The use of a new 3D splint and double CT scan procedure to obtain an accurate anatomic virtual augmented model of the skull", INTERNATIONAL JOURNAL OF ORAL AND MAXILLOFACIAL SURGERY, COPENHAGEN, DK, vol. 36, no. 2, 3 February 2007 (2007-02-03), pages 146 - 152, XP005866236, ISSN: 0901-5027, DOI: 10.1016/J.IJOM.2006.09.019
- GWEN R. J. SWENNEN ET AL: "A Cone-Beam Computed Tomography Triple Scan Procedure to Obtain a Three-Dimensional Augmented Virtual Skull Model Appropriate for Orthognathic Surgery Planning :", THE JOURNAL OF CRANIOFACIAL SURGERY, vol. 20, no. 2, 1 March 2009 (2009-03-01), US, pages 297 - 307, XP055687608, ISSN: 1049-2275, DOI: 10.1097/SCS.0b013e3181996803
- KANG S-H ET AL: "Dental image replacement on cone beam computed tomography with three-dimensional optical scanning of a dental cast, occlusal bite, or bite tray impression", INTERNATIONAL JOURNAL OF ORAL AND MAXILLOFACIAL SURGERY, COPENHAGEN, DK, vol. 43, no. 10, 9 July 2014 (2014-07-09), pages 1293 - 1301, XP029061663, ISSN: 0901-5027, DOI: 10.1016/J.IJOM.2014.06.009

## Description

### Object of the invention

The present invention relates to a method for designing a dental prosthesis using digital techniques which can later be manufactured in a numerical control machine. The method object of the invention can be applied to the dental implantology industry, and specifically to the industry of manufacturing dental prostheses.

### Technical problem to be solved and background of the invention

Taking impressions using elastomeric resin has been common practice for obtaining the information of a patient's mouth. A tray with the shape and dimensions of a patient's dental arch is filled with this resin and it is introduced in the mouth, covering it entirely. The resin is left for a few minutes in the patient's mouth in order to solidify and then the definitive impression is extracted.

If there are implants, the impression is begun by placing posts screwed into the implants and trimming down the impression tray so said posts do not come into contact with it. The resin is then added around the implants, the tray is filled with material and it is placed on the working area, allowing the resin to fill in all of the gaps. To finish the process, once it has solidified, the posts in place are unscrewed and the impression is removed. And lastly, the analogs are screwed into the impression and the information is sent to the laboratory.

The dental prosthesis laboratory uses this information to begin the manufacturing process of the plaster casts by forming a positive of the dental impression taken.

The plaster casts are scanned using fixed scanners in the laboratory. Although the precision of the scanner is considered satisfactory, the manufacturing process of the plaster cast can lead to errors in the impression of the cast with the elastomeric resin, which can become deformed while taking the impression or when transporting it from the clinic to the laboratory, or in the subsequent manufacturing of the plaster cast, due to the contraction in the hardening thereof that can vary depending on the cast proportions and water used, which makes this part of the process susceptible to errors.

To validate the data obtained for the manufacturing of the prosthesis on implants, a passivity test is used.

This passivity test aims to manufacture a self-polymerising resin structure that joins all of the posts to the implants. This structure is divided into sections and sent to the clinic. At the clinic, each section is placed on its corresponding implant in the patient's mouth and they are joined, thereby capturing the position of the implants.

The modified structure is transferred to the plaster cast. The implant analogs are placed on the posts fixed to the structure, except on one of them, to which the respective implant in the cast is screwed. In order for the rest of the implants to fit in the model, a hole is opened in the cast in the approximate position where the implant analog is located. The corresponding post is then screwed in and the gaps between the cast and the analog situated in the passivized structures are filled in.

By correctly executing this process, which combines non-digital and digital steps, a proper adjustment can be achieved, although it is a burdensome and costly process that additionally requires meeting with the patient to test the structures in their mouth. Passivized plaster casts are scanned with a structured-light optical scanner in the laboratory, creating a digitalised cast.

The scans to be done with the laboratory scanners are of the areas that need to be restored: the gums, the scanning posts, the prior aesthetic simulations and the occlusion therebetween. All of the scans are done separately and will later be aligned with one another using a known algorithm.

At this time the position of the implants is virtually located. Points are drawn that relate the scanning post to a virtual post in order to position the implant based on the position of the same. At this time, the design of the dental prosthesis starts.

There is also a digitalised method in the state of the art for making definitive prosthesis designs, which starts by directly scanning the patient's mouth using an intraoral structured-light 3D scanner.

In the intraoral scans of implants to digitally locate the implants, the same method for placing the scanning posts that is done for plaster casts in the laboratory is carried out, placing and then scanning the scanning posts.

For completely edentulous patients, the only current option for scanning the occlusion and establishing a correct vertical dimension, which is the space as well as the way of articulating between the jaws, is by the use of previously screwed provisional posts. If they are present, the method established by the manufacturer would be:
1. scanning the lower jaw and the upper jaw with previously positioned provisional teeth and/or remnant teeth.
2. scanning the occlusion between provisional teeth.
3. trimming the provisional teeth, leaving as much of the gums as possible.
4. scanning the tissues in the trimmed model that were not able to be scanned due to the fact that the provisional was in place.
5. scanning the scanning posts over the scan of the tissues.

There is a problem with these types of scanners when coming across transparent or highly reflexive surfaces, since light does not behave the same in completely opaque areas. In certain types of patients, mainly in edentulous patients, in which there are no reference elements and all areas are covered by a thin layer of saliva, there may be deviations in the scanned files due to an incorrect triangulation.

A special camera has been developed, which has been effectively tested to capture the positions of the patient's implants regardless of how many implants there are in their dental arch, their position, distances or angulation.

This special camera uses photogrammetry for digitalization, which is the science of taking measurements from photography, especially for knowing the exact position of the points on a surface.

The digitalisation process consists of the placement of the scanning posts, which are flag-shaped elements with four points, on each of the patient's implants.

Using the special camera, we take a large number of pictures of the scanning posts from different positions. These images are then processed using a triangulation algorithm, which converts two-dimensional images into a point cloud, which recognises the position of the scanning posts and digitalises them, obtaining only a point cloud or mesh in which the relative distance and angulation between implants are provided. This information is combined with the scan of the tissues, from which the scan of the tissues with the passivized implants is obtained. This passivation can be done on the intraoral scans or on scans from plaster casts.

This system requires additional tests to be done in the patient's mouth and a special camera that is only used for this operation. Furthermore, there is only correction of the relative position between implants and not of the deformation that may exist between tissues.

It is also known the study shown in the article *"*Dental image replacement on cone beam computed tomography with three-dimensional optical scanning of a dental cast, occlusal bite, or bite tray impression" of Kang S-H et al, published on July 9, 2014 in the vol.43, no. 10 of the International Journal of Oral and Maxillofacial Surgery, whose goal is to compare the accuracy of dental image replacement on a Cone Beam Computed Tomography (CBCT) image using digital image data from three-dimensional (3D) optical scanning of three experimental materials: a dental cast, occlusal bite, and bite tray impression. A Bracket Typodont dental model was used. CBCT of the dental model was performed and the data were converted to stereolithography (STL) format. STL files converted from the CBCT of the Typodont model and the 3D optical-scanned STL files of the experimental materials were image-registered. The error range of each methodology was measured and compared with 3D optical scan of the Typodont. The specific aim of this study is to verify if there is any deviation between the three different experimental material scans and the respective CBCT scans. That is, the study is limited to detecting deviations between both scanning techniques (optical scan and CBCT scan), but the deviations of the optical scan are not corrected with CBCT scan. The deviations between the STLs are simply detected and recorded, without actually taking any corrective action that generates a benefit from the use of the CBCT scan.

### Description of the invention

The object of the invention is a method for designing a dental prosthesis using digital techniques that comprises the following steps: scanning tissues of the upper jaw, via an intraoral digital scan, thereby obtaining a mesh representation of the tissue of the upper jaw, scanning tissues of the lower jaw, via the intraoral digital scan, thereby obtaining a mesh of the tissue of the lower jaw, scanning some scanning posts placed in respective implants, via the intraoral digital scan, thereby obtaining a scanned mesh of posts for each of the dental arches to be restored, from which the relative position between implants is obtained, and scanning the occlusion of the jaws, via the intraoral digital scan, thereby obtaining an occlusion mesh.

The method object of the invention comprises the following additional steps to verify that the information obtained in the step of scanning the scanning posts is correct:
- obtaining a digitally treated mesh of posts for each jaw by means of the following steps:
   - obtaining a second mesh with the scanning posts placed in the implants by means of computed tomography (CT) or cone beam computed tomography (CBCT);
   - generating three-dimensional files for two established density ranges by processing the second mesh obtained from the previous step in a DICOM file viewer software, obtaining a first three-dimensional file for the scanning posts made of a first material and a second three-dimensional file for the implants made of a second material;
   - exporting the first and second three-dimensional files to respectively CAD files;
   - removing from the first and second three-dimensional files, using a CAD software, all of the elements that are not scanning posts or implants respectively;
   - combining respective information of the scanning posts and implants of the first and second three-dimensional files into a single mesh, obtaining the digitally treated mesh of posts;
   - adapting the scanned mesh of posts for each jaw to the respective digitally treated mesh of posts by means of the following steps:
      - placing virtual scanning posts on the scanning posts of the digitally treated mesh of posts,
      - aligning the virtual scanning posts with the direction and position of the implants of the digitally treated mesh of posts,
      - adapting the position of the scanning posts of the scanned mesh of posts to the position of the virtual scanning posts that have already been aligned, this adaptation being done by means of the following steps:
         o dividing the jaw by a mid-line into a first half and a second half of jaw,
         o making the posts of the first jaw half of the scanned mesh of posts coincide with the corresponding virtual scanning posts previously aligned,
         o making the posts of the second jaw half of the scanned mesh of posts coincide with the corresponding virtual scanning posts previously aligned,
         o filling in a step created between the first jaw half and the second jaw half of the scanned mesh of posts with a smooth transformation,
         o removing the scanning posts from the scanned mesh of posts, obtaining a third mesh with the aligned virtual scanning posts and the gum of the scanned mesh of posts,
      - joining, in the third mesh obtained, the virtual scanning posts to the gum.

- fusing the previously obtained meshes into a final model of the digital structure of the dental prosthesis, via a dental design CAD software.

If a patient does not have remnant teeth, the method for designing a dental prosthesis using digital techniques may comprise the following additional steps:
- verifying that the information obtained in the step of scanning the occlusion is correct:
   - if the patient has scanning posts or has a provisional screwed in, the scanning of the occlusion is done conventionally, with a scanner that provides correct information;
   - if the patient has removable teeth, the following steps are carried out:
      - scanning the two removable sets of teeth to obtain a mesh of each removable set of teeth;
      - aligning the meshes of each removable set of teeth using conventional methods in the occlusion position;
      - obtaining a single mesh that reflects the relative position between the surfaces of the two removable sets of teeth that adapt to the gum, and to do so the following steps are carried out:
         - carrying out a digital edition of the meshes of the removable set of teeth in a CAD software;
         - combining the meshes of each removable set of teeth using virtual combination tools to obtain a single mesh;
         - eliminating the surfaces that are not adapted to the gums of the jaws; and
         - inverting the mesh that reflects only the relative position between the surfaces of the two removable sets of teeth that adapt to the gum, thereby obtaining the occlusion mesh.

The method for designing a dental prosthesis using digital techniques may comprise the additional step of applying a finite element analysis in order to know the mechanical loads in each of the elements of the digital structure of the prosthesis, and if there is any weak point or area in the structure of the prosthesis, the weak point or area is reinforced.

Lastly, the method for designing a dental prosthesis using digital techniques may comprise the step of applying a second finite element analysis to check the stresses tolerated by the structure.

### Description of the drawings

To complete the description, and for the purpose of helping to make the features of the invention more readily understandable, the present specification is accompanied by a set of figures constituting an integral part of the same, which by way of illustration and not limitation represent the following:

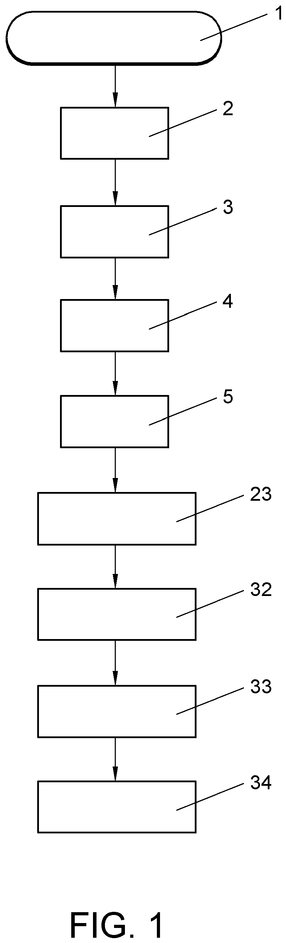

Figure 1 shows a block diagram of the method for designing a dental prosthesis using digital techniques object of the invention.

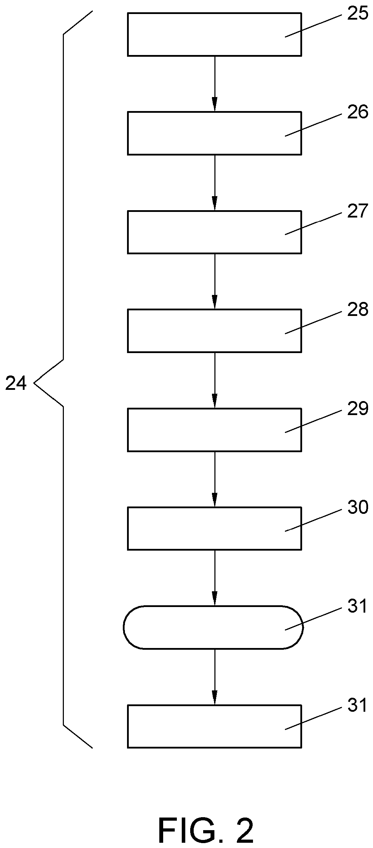

Figure 2 shows a block diagram of the method for scanning an occlusion in the case that the patient has removable teeth.

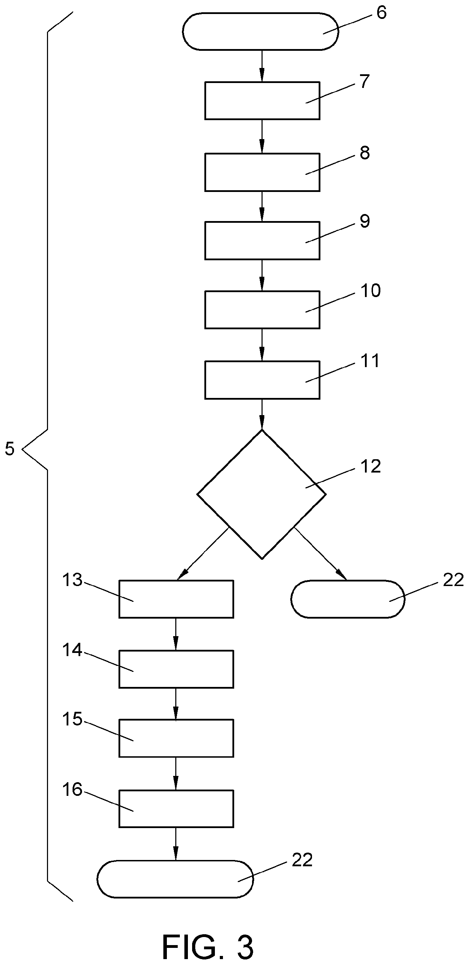

Figure 3 shows a block diagram of the steps of verifying that the information obtained in the step of scanning the scanning posts is correct.

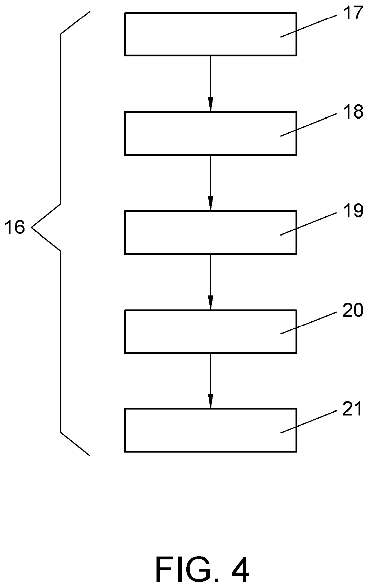

Figure 4 shows a block diagram of the steps for adapting the scanned mesh of posts to the digitally treated mesh of posts used in the method for designing a dental prosthesis using digital techniques object of the invention.

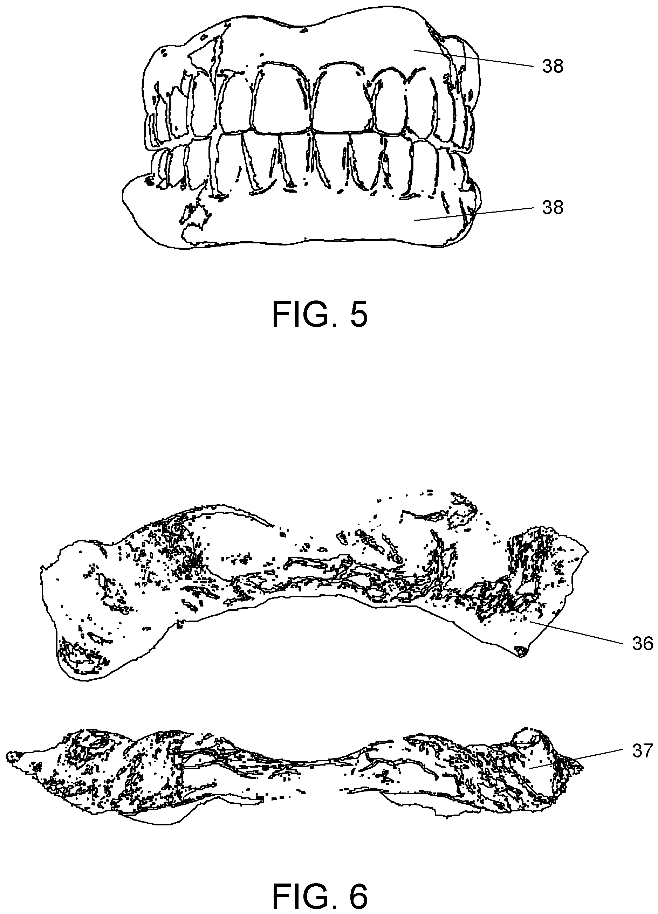

Figure 5 shows a perspective view of a scan of the two removable sets of teeth.
Figure 6 shows a perspective view of the scanned mesh showing the surface of two facing gums.

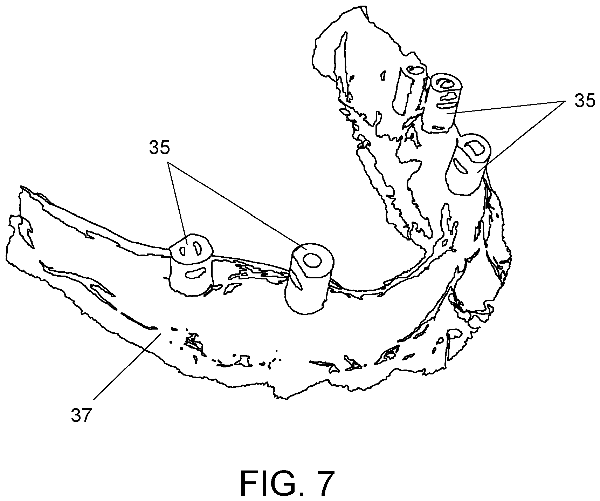

Figure 7 shows a perspective view of a jaw with the scanning posts.

### Description of an embodiment of the invention

A description of an exemplary embodiment of the method object of the invention is provided below, including references to the figures.

The method for designing a dental prosthesis using digital techniques comprises the following steps:
- scanning the upper jaw (1), thereby obtaining a mesh representation of the upper jaw (36);
- scanning the lower jaw (2), thereby obtaining a mesh of the lower jaw (37);
- scanning some scanning posts (35) placed in respective implants of each jaw, thereby obtaining a scanned mesh of posts from which the relative position between implants (3) is obtained;
- scanning the occlusion of the jaws (4), obtaining at least one occlusion mesh.

The aforementioned steps can be carried out on a plaster cast taken from the patient's mouth or directly on the patient's mouth using an intraoral scanner.

It is possible for the meshes of both the upper jaw and the lower jaw to be only the tissues of said jaws in the case that a patient does not have remnant teeth, or they may be the tissues of said jaws and of the remnant pieces.

In patients who have remnant teeth, with these four steps, we would have all the necessary information in order to design the dental prosthesis, however, there is a possibility that the relative position between the implants obtained by scanning the scanning posts (35) is incorrect, and thus in the method object of the invention a series of verifications is subsequently carried out, which will be explained further on in this specification, in order to verify and correct the information of the relative position between implants.

In patients who do not have remnant teeth, it is necessary to check the relative position between implants and the information obtained in the step of scanning the occlusion, such that the method for designing a dental prosthesis using digital techniques may include a series of verifications to prevent errors in the relative position between implants and also involves verifying the information of the scan of the occlusion.

In the aforementioned case, in which the patient does not have remnant teeth, it may be verified that the information obtained in the step of scanning the occlusion is correct (24) using a method that varies depending on the elements the patient has in their mouth. In this case, the method depends on whether the patient has scanning posts (35), a provisional screwed in, or removable teeth (38).

In the first two cases (the patient has scanning posts (35) or has a provisional screwed in), the scanning of the occlusion may be done conventionally, with a scanner that provides correct information.

In the case that the patient has removable teeth (38), the method for scanning an occlusion may consist of the following steps:
- scanning the two removable sets of teeth (38) to obtain a mesh of each removable set of teeth (25);
- aligning the meshes of each removable set of teeth (26) using conventional methods;
- obtaining a single mesh that only reflects the relative position between the surfaces of the two removable sets of teeth (38) that adapt to the gum (27), and to do so, a digital edition of the mesh of the removable teeth (28) is carried out by using a CAD software, and
   the meshes of each removable set of teeth are combined using virtual combination tools to obtain a single mesh (29);
- eliminating the surfaces that are not adapted to the gums of the jaws (30);
- inverting the mesh that reflects only the relative position between the surfaces of the two removable sets of teeth (38) that adapt to the gum (31), thereby obtaining the occlusion mesh;
- putting the scan of the upper and lower jaw with respect to the occlusion obtained into a dental design CAD software, thereby ensuring the correct occlusion of the dental prosthesis to be manufactured.

Likewise, the method verifies that the information obtained in the step of scanning the scanning posts is correct (5), in other words, it verifies that the relative position between implants is correct.

To verify that the information obtained in the step of scanning the scanning posts is correct, firstly a digitally treated mesh of posts (6) is obtained, and to obtain this digitally treated mesh of posts, the following steps are carried out:
- obtaining a second mesh of posts with the relative positions between the implants by means of computed tomography (CT) or cone beam computed tomography (CBCT) (7) with the scanning posts (35) placed in the implants of the jaws, which basically consists of taking three-dimensional X-rays of the jaws with the scanning posts (35) placed in the implants of the jaws;
- processing the digital information (the second mesh of posts) obtained in a DICOM file viewer software, generating three-dimensional files for two established density ranges (8), thereby obtaining a first three-dimensional file for the head of the scanning posts (35) that are made of a first material and a second three-dimensional file for the implants that are made of a second material;
- exporting the first and second three-dimensional files to respectively CAD files (9);
- using a CAD software, the first and second three-dimensional files are cleaned up, eliminating all elements that are neither the scanning posts nor the implants (10) respectively;
- combining respective information of the scanning posts (35) and implants (11) of the first and second three-dimensional files into a single mesh, obtaining the digitally treated mesh of posts.

The step of obtaining relative positions between implants, by means of computed tomography (CT) or cone beam computed tomography (CBCT) is carried out with the scanning posts (35) placed in the implants of the jaws to facilitate the treatment process of the meshes and to have more reference elements that define the height and axis of the implant, but in another embodiment, which does not belong to the invention, the same result could be achieved by doing computed tomography (CT) or cone beam computed tomography (CBCT) exclusively of the implants.

The density ranges for the three-dimensional files are defined by the different densities of the scanning posts (35) and the implants.

The coincidence or discrepancy between the initial scan of the scanning posts (35) (scanned mesh of posts) and the second mesh obtained through the tomography of the aforementioned posts (digitally treated mesh of posts) (12) is then verified, and to do so, the posts of one side of the jaw are aligned and the deviation on the opposite side is observed.

If the scanned mesh of posts and the digitally treated mesh of posts coincide, embodiment not belonging to the invention, the scanned mesh of posts is considered correct and a design may be done on it.

If the two meshes do not coincide, it is necessary to adapt the scanned mesh of posts to the digitally treated mesh of posts (13).

To adapt the scanned mesh of posts to the digitally treated mesh of posts (13), virtual scanning posts are used, which are elements used to virtually place the scanning posts (35) on each of the implants of the digitally treated mesh of posts to correct the scanned mesh. The virtual scanning posts consist of a mesh formed by a head with the shape of the scanning posts scanned along with the gum and an axis concentric to the head with a length greater than or equal to the implant.

The adaptation is carried out in the following way:
- virtual scanning posts are placed on the scanning posts (35) of the digitally treated mesh of posts (14),
- the virtual scanning posts are aligned with the direction of the implants of the digitally treated mesh of posts (15),
- the position of the scanning posts (35) of the scanned mesh of posts is adapted to the position of the virtual scanning posts that have already been aligned (16), and this adaptation is done by means of the following steps:
   - dividing the jaw by the mid-line into a first half and a second half of jaw (17),
   - on the first half of jaw, making the posts of the scanned mesh of posts coincide with the corresponding virtual scanning posts previously aligned (18),
   - on the second half of jaw, making the posts of the scanned mesh of posts coincide with the corresponding virtual scanning posts previously aligned (19),
   - between the first half and the second half of jaw of scanned mesh of posts, a step is created that is filled in with a smooth transformation (20),
   - removing the scanning posts (35) from the scanned mesh of posts, obtaining a third mesh with the aligned virtual scanning posts and the gum of the scanned mesh of posts (21),
   - joining, in the third mesh obtained, the virtual scanning posts (35) to the gum (22).

By the aforementioned adaptation, a mesh is obtained that faithfully reflects the surface of the gums of the two jaws, the relative position of the two jaws is verified and the implants in the jaws are in a position that has been verified to be exact. Based on this mesh and the other previously obtained, in the dental design CAD software, the definitive digital structure of the prosthesis (23) is obtained.

Once the digital structure of the prosthesis is obtained, the aforementioned digital structure may be introduced into a software in order to apply a finite element analysis (32), which allows the mechanical loads of each of the elements of the digital structure of the prosthesis to be known, such that we can know beforehand if there is any weak point or area in the structure of the prosthesis, meaning an area or point that is going to be subjected to a load greater than that which the material can withstand. Thus, if there is any weak point or area in the structure of the prosthesis, these weak points or areas are reinforced (33).

Once the necessary reinforcements for the weak areas have been established, a second finite element analysis may be carried out to check the stresses tolerated by the structure (34).

Once it has been checked that the stresses of the structure are admissible, the dental prosthesis is manufactured using a numerical control machine, and then finished by a prosthetic technician.

## Claims

1. A method for designing a dental prosthesis using digital techniques comprising the following steps:
- scanning an upper jaw, via an intraoral digital scan, thereby obtaining a mesh representation of the upper jaw (1);
- scanning a lower jaw, via the intraoral digital scan, thereby obtaining a mesh of the lower jaw (2);
- scanning some scanning posts (35) placed in respective implants of each jaw, which marking a direction and a position of each implant, via the intraoral digital scan, thereby obtaining a scanned mesh of posts for each jaw, from which the relative position between implants (3) is obtained;
- scanning an occlusion of the jaws (4), via the intraoral digital scan, thereby obtaining an occlusion mesh;
**characterised in that** it comprises the additional steps of:
- obtaining a digitally treated mesh of posts for each jaw (6) by means of the following steps:
• obtaining a second mesh with the scanning posts (35) placed in the implants by means of computed tomography (CT) or cone beam computed tomography (CBCT) (7);
• generating three-dimensional files for two established density ranges (8) by processing the second mesh obtained from the previous step in a DICOM file viewer software, obtaining a first three-dimensional file for the scanning posts (35) made of a first material and a second three-dimensional file for the implants made of a second material;
• exporting the first and second three-dimensional files to respectively CAD files (9);
• removing from the first and second three-dimensional files, using a CAD software, all of the elements that are not scanning posts or implants (10) respectively;
• combining respective information of the scanning posts (35) and implants (11) of the first and second three-dimensional files into a single mesh, obtaining the digitally treated mesh of posts;
- adapting the scanned mesh of posts for each jaw to the respective digitally treated mesh of posts (13) by means of the following steps:
• placing virtual scanning posts on the scanning posts (35) of the digitally treated mesh of posts (14),
• aligning the virtual scanning posts with the direction and position of the implants of the digitally treated mesh of posts (15),
• adapting the position of the scanning posts (35) of the scanned mesh of posts to the position of the virtual scanning posts that have already been aligned (16), this adaptation being done by means of the following steps:
∘ dividing the jaw by a mid-line into a first half and a second half of jaw (17),
∘ making the posts of the first jaw half of the scanned mesh of posts coincide with the corresponding virtual scanning posts previously aligned (18),
∘ making the posts of the second jaw half of the scanned mesh of posts coincide with the corresponding virtual scanning posts previously aligned (19),
∘ filling in a step created between the first jaw half and the second jaw half of the scanned mesh of posts with a smooth transformation (20),
∘ removing the scanning posts from the scanned mesh of posts, obtaining a third mesh with the aligned virtual scanning posts and a gum of the scanned mesh of posts (21),
∘ joining, in the third mesh obtained, the virtual scanning posts to the gum (22);
- fusing the previously obtained meshes (23) into a final model of the digital structure of the dental prosthesis, via a dental design CAD software.

2. The method for designing a dental prosthesis using digital techniques according to the claim 1, **characterised in that** it comprises the following additional step:
- applying a finite element analysis (32) in order to know the mechanical loads in each of the elements of the digital structure of the prosthesis,
- if there is any weak point or area in the structure of the prosthesis, the weak point or area is reinforced (33).

3. The method for designing a dental prosthesis using digital techniques according to claim 2, **characterised in that** it comprises the step of applying a second finite element analysis (34) to check the stresses tolerated by the structure.

## Patentansprüche

1. Verfahren zum Entwerfen einer Zahnprothese mittels digitaler Techniken, das die folgenden Schritte umfasst:
- Scannen eines Oberkiefers mittels eines digitalen Intraoralscans, wodurch eine Netzdarstellung des Oberkiefers (1) erhalten wird;
- Scannen eines Unterkiefers mittels des digitalen Intraoralscans, wodurch ein Netz des Unterkiefers (2) erhalten wird;
- Scannen einiger Scanpfosten (35), die in den jeweiligen Implantaten jedes Kiefers eingesetzt sind und eine Richtung und eine Position jedes Implantats markieren, mittels des digitalen Intraoralscans, wodurch ein gescanntes Netz von Pfosten für jeden Kiefer erhalten wird, aus dem die relative Position zwischen den Implantaten (3) ermittelt wird;
- Scannen einer Okklusion der Kiefer (4) mittels des digitalen Intraoralscans, wodurch ein Okklusionsnetz erhalten wird;
**dadurch gekennzeichnet, dass** es die folgenden zusätzlichen Schritte umfasst:
- Erhalten eines digital aufbereiteten Pfostennetzes für jeden Kiefer (6) mittels der folgenden Schritte:
• Erhalten eines zweiten Netzes mit den in den Implantaten platzierten Scanpfosten (35) mittels Computertomographie (CT) oder Cone-Beam-Computertomographie (CBCT) (7);
• Generieren dreidimensionaler Dateien für zwei festgelegte Dichtebereiche (8) durch Verarbeiten des im vorherigen Schritt erhaltenen zweiten Netzes in einer DICOM-Dateibetrachtersoftware, wodurch eine erste dreidimensionale Datei für die Scanpfosten (35) aus einem ersten Material und eine zweite dreidimensionale Datei für die Implantate aus einem zweiten Material erhalten wird;
• Exportieren der ersten und zweiten dreidimensionalen Datei in jeweilige CAD-Dateien (9);
• Entfernen aller Elemente aus der ersten und zweiten dreidimensionalen Datei mithilfe einer CAD-Software, bei denen es sich nicht um Scanpfosten bzw. Implantate (10) handelt;
• Kombinieren der jeweiligen Informationen der Scanpfosten (35) und Implantate (11) der ersten und zweiten dreidimensionalen Datei in einem einzigen Netz, wodurch das digital aufbereitete Pfostennetz erhalten wird;
- Anpassen des gescannten Pfostennetzes für jeden Kiefer an das jeweils digital aufbereitete Pfostennetz (13) mittels folgender Schritte:
• Platzieren von virtuellen Scanpfosten auf die Scanpfosten (35) des digital aufbereiteten Pfostennetzes (14),
• Ausrichten der virtuellen Scanpfosten mit der Richtung und Position der Implantate des digital aufbereiteten Pfostennetzes (15),
• Anpassen der Position der Scanpfosten (35) des gescannten Pfostennetzes an die Position der bereits ausgerichteten virtuellen Scanpfosten (16), wobei diese Anpassung mittels der folgenden Schritte erfolgt:
∘ Unterteilen des Kiefers durch eine Mittellinie in eine erste und eine zweite Kieferhälfte (17),
∘ Herstellen einer Übereinstimmung der Pfosten der ersten Kieferhälfte des gescannten Pfostennetzes mit den entsprechenden, zuvor ausgerichteten virtuellen Scanpfosten (18),
∘ Herstellen einer Übereinstimmung der Pfosten der zweiten Kieferhälfte des gescannten Pfostennetzes mit den entsprechenden, zuvor ausgerichteten virtuellen Scanpfosten (19),
o Ausfüllen einer zwischen der ersten Kieferhälfte und der zweiten Kieferhälfte des gescannten Pfostennetzes entstandenen Stufe mit einer sanften Transformation (20),
∘ Entfernen der Scanpfosten aus dem gescannten Pfostennetz, Erhalten eines dritten Netzes mit den ausgerichteten virtuellen Scanpfosten und eines Zahnfleischs des gescannten Pfostennetzes (21),
∘ Verbinden der virtuellen Scanpfosten mit dem Zahnfleisch (22) im dritten erhaltenen Netz;
- Zusammenführen der zuvor erhaltenen Netze (23) zu einem endgültigen Modell der digitalen Struktur der Zahnprothese mittels einer Dentaldesign-CAD-Software.

2. Verfahren zum Entwerfen einer Zahnprothese mittels digitaler Techniken nach Anspruch 1, **dadurch gekennzeichnet, dass** es den folgenden zusätzlichen Schritt umfasst:
- Anwenden einer Finite-Elemente-Analyse (32), um die mechanischen Belastungen in jedem der Elemente der digitalen Struktur der Prothese zu ermitteln,
- Wenn es in der Struktur der Prothese Schwachstellen oder Bereiche gibt, werden diese verstärkt (33).

3. Verfahren zum Entwerfen einer Zahnprothese mittels digitaler Techniken nach Anspruch 2, **dadurch gekennzeichnet, dass** es den Schritt des Anwendens einer zweiten Finite-Elemente-Analyse (34) umfasst, um die von der Struktur tolerierten Spannungen zu überprüfen.

## Revendications

1. Procédé de conception d'une prothèse dentaire au moyen de techniques numériques comprenant les étapes suivantes :
- le balayage d'une mâchoire supérieure, via un balayage numérique intraoral, donnant ainsi une représentation en maillage de la mâchoire supérieure (1) ;
- le balayage d'une mâchoire inférieure, via le balayage numérique intraoral, donnant ainsi un maillage de la mâchoire inférieure (2) ;
- le balayage de certains corps de balayage (35) placés dans des implants respectifs de chaque mâchoire, qui marquent une direction et une position de chaque implant, via le balayage numérique intraoral, donnant ainsi un maillage de corps balayé pour chaque mâchoire, à partir duquel la position relative entre les implants (3) est obtenue ;
- le balayage d'une occlusion des mâchoires (4), via le balayage numérique intraoral, donnant ainsi un maillage d'occlusion ;
**caractérisé en ce qu'**il comprend les étapes supplémentaires suivantes :
- l'obtention d'un maillage de corps traité numériquement pour chaque mâchoire (6) au moyen des étapes suivantes :
• l'obtention d'un deuxième maillage avec les corps de balayage (35) placés dans les implants au moyen d'une tomodensitométrie (TDM) ou d'une tomodensitométrie volumétrique à faisceau conique (TVFC) (7) ;
• la génération de fichiers tridimensionnels pour deux plages de densité établies (8) en traitant le deuxième maillage obtenu à l'étape précédente dans un logiciel de visualisation de fichiers DICOM, l'obtention d'un premier fichier tridimensionnel pour les corps de balayage (35) constitués d'un premier matériau et d'un second fichier tridimensionnel pour les implants constitués d'un second matériau ;
• l'export des premier et second fichiers tridimensionnels vers des fichiers de CAO (9) respectifs ;
• le retrait, à partir des premier et second fichiers tridimensionnels, à l'aide d'un logiciel de CAO, de tous les éléments qui ne sont pas respectivement des corps de balayage ou des implants (10) ;
• la combinaison d'informations respectives des corps de balayage (35) et des implants (11) des premier et second fichiers tridimensionnels en un seul maillage, donnant ainsi le maillage de corps traité numériquement ;
- l'adaptation du maillage de corps numérisé de chaque mâchoire au maillage respectif de corps (13) traité numériquement au moyen des étapes suivantes :
• le placement de corps de balayage virtuels sur les corps de balayage (35) du maillage de corps (14) traité numériquement,
• l'alignement des corps de balayage virtuels avec la direction et la position des implants du maillage de corps (15) traité numériquement,
• l'adaptation de la position des corps de balayage (35) du maillage de corps balayé à la position des corps de balayage virtuels déjà alignés (16), cette adaptation étant réalisée au moyen des étapes suivantes :
∘ la division de la mâchoire à l'aide d'une ligne médiane, en une première moitié et une seconde moitié de mâchoire (17),
∘ le fait de faire coïncider les corps de la première moitié de mâchoire du maillage de corps balayé avec les corps de balayage virtuels (18) correspondants préalablement alignés,
∘ le fait de faire coïncider les corps de la seconde moitié de mâchoire du maillage de corps balayé avec les corps de balayage virtuels (19) correspondants préalablement alignés,
∘ le remplissage d'un espace créé entre la première moitié de mâchoire et la seconde moitié de mâchoire du maillage de corps numérisé avec une transformation lisse (20),
∘ le retrait des corps de balayage du maillage de corps balayé, l'obtention d'un troisième maillage avec les corps de balayage virtuels alignés et une gencive du maillage de corps balayé (21),
∘ la jonction, dans le troisième maillage obtenu, des corps de balayage virtuels à la gencive (22) ;
- la fusion des maillages (23) précédemment obtenus dans un modèle final de la structure numérique de la prothèse dentaire, via un logiciel de CAO de conception dentaire.

2. Procédé de conception d'une prothèse dentaire à l'aide de techniques numériques selon la revendication 1, **caractérisé en ce qu'**il comprend l'étape supplémentaire suivante :
- l'application d'une analyse par éléments finis (32) afin de connaître les charges mécaniques dans chacun des éléments de la structure numérique de la prothèse,
- s'il existe un point ou une zone faible quelconque dans la structure de la prothèse, le point ou la zone faible est renforcé (33).

3. Procédé de conception d'une prothèse dentaire à l'aide de techniques numériques selon la revendication 2, **caractérisé en ce qu'**il comprend l'étape d'application d'une seconde analyse par éléments finis (34) pour vérifier les contraintes tolérées par la structure.
